# FASCICULE DE BREVET EUROPEEN

(11) **EP 1 269 988 B1**
(45) Date de publication et mention de la délivrance du brevet: **05.12.2007**
(21) Numéro de dépôt: 02291596.1
(22) Date de dépôt: 26.06.2002
(51) Int. Cl.: A61K 8/34, A61K 8/42, A61K 8/44, A61K 8/49, A61Q 7/00, A61Q 19/00, A61Q 19/08

(54) **Composition cosmetique ou dermatologique comprenant des dérivés d'acylaminoamide**
Acylaminoamid-Derivate enthaltende kosmetische oder dermatologische Zusammensetzung
Cosmetic or dermatological composition comprising acylaminoamide derivatives

(30) Priorité: 26.06.2001 FR 0108432
(43) Date de publication de la demande: 02.01.2003
(73) Titulaire: L'ORÉAL, 75008 Paris (FR)
(72) Inventeur: Breton, Lionel, 78000 Versailles (FR); Mahe, Yann, 91390 Morsang sur Orge (FR)
(74) Mandataire: Catherine, Alain

(56) Documents cités:
- EP-A- 0 909 557
- WO-A-00/12467
- DE-A- 2 322 232
- FR-A- 2 810 033
- US-A- 5 234 909
- PATENT ABSTRACTS OF JAPAN vol. 015, no. 001 (C-0793), 7 janvier 1991 (1991-01-07) & JP 02 256609 A (KENJI ICHIKAWA;OTHERS: 01), 17 octobre 1990 (1990-10-17)
- NAKAMURA M ET AL: "A two-step, one-pot synthesis of diverse N-pyruvoyl amino acid derivatives using the Ugi reaction" BIOORGANIC & MEDICINAL CHEMISTRY LETTERS, OXFORD, GB, vol. 10, no. 24, 18 décembre 2000 (2000-12-18), pages 2807-2810, XP004225356 ISSN: 0960-894X

## Description

La présente invention se rapporte au domaine des compositions cosmétiques ou dermatologiques. Elle est relative à de nouvelles compositions cosmétiques ou dermatologiques comprenant une association entre un composé inhibiteur de l'élastase de la famille des N-Acylaminoamides et au moins un agent antifongique ou au moins un agent antibactérien. Cette composition est préférentiellement destinée à améliorer les signes cutanés du vieillissement et/ou du photovieillissement en ralentissant les altérations du tissu conjonctif et en améliorant l'état fonctionnel de la peau tout en contrôlant l'excès de bactéries et/ou de levures résidentes et/ou pathogènes.

La peau humaine est constituée de deux compartiments à savoir un compartiment superficiel, l'épiderme, et un compartiment profond, le derme. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses.

On sait que lors d'un stress cutané superficiel, qui peut notamment être d'origine chimique, physique ou bactérienne, les kératinocytes des couches superficielles de l'épiderme libèrent des médiateurs biologiques qui possèdent la capacité d'attirer certaines cellules infiltrantes de la peau, elles-mêmes responsables de l'entretien d'une irritation locale transitoire.

Parmi les médiateurs biologiques pouvant être produits par les kératinocytes ainsi stressés, on citera les chimiokines qui sont des cytokines chimioattractives responsables du recrutement de leucocytes sur les sites inflammatoires, dont l'interleukine 8 (IL-8) qui est plus particulièrement responsable du recrutement des neutrophiles.

Ces cellules infiltrant les zones irritées ou agressées libèrent alors des enzymes parmi lesquelles on peut citer l'élastase leucocytaire.
Sous l'action de cette enzyme notamment, les fibres élastiques de soutien extracellulaire du tissu conjonctif peuvent être dégradées, et entraîner ainsi une diminution de l'élasticité de la peau.

Il est même par ailleurs connu qu'en synergie avec la cathepsine G, l'élastase leucocytaire peut dissocier l'intégrité de l'épiderme en élargissant les espaces intercellulaires interkératinocytaires.

Ainsi, à long terme, la somme des micro-stress cutanés superficiels, par exemple générés par une exposition prolongée aux UV ou par des agents irritants, peut entraîner une perte plus ou moins accélérée de l'élasticité naturelle de la peau. Le réseau formé par les fibres élastiques du tissu conjonctif sous-jacent et des espaces extracellulaires peut alors progressivement être destructuré. Il s'en suit un vieillissement accéléré de la peau (peau ridée et/ou moins souple) via l'altération du réseau élastique dermique, ainsi qu'une accentuation des rides (rides plus profondes).

Sous l'action de cette enzyme, les fibres élastiques de soutien extracellulaire du tissu conjonctif sont dégradées. En synergie avec la cathepsine G, l'élastase leucocytaire peut même dissocier l'intégrité de l'épiderme en élargissant les espaces intercellulaires inter-kératinocytaires (Ludolph-Hauser et al Exp. Dermatol. 1999 8(1) 46-52). L'élastase leucocytaire a récemment été incriminée dans l'entretien des escarres et la survenue des ulcères veineux des jambes, via son activité de dégradation de la fibronectine (Herrick S et al Lab.Invest. 1997(3) 281-288). La somme des agressions induites par des micro-stress localisés (suite par exemple à une exposition prolongée au soleil) peut aboutir au long terme à une perte accélérée de l'élasticité naturelle de la peau. Le réseau des fibres élastiques du tissu conjonctif sous jacent et des espaces extracellulaires est alors progressivement déstructuré. Cette dégradation accélérée peut se cumuler avec le processus de vieillissement normal de la peau qui se caractérise par une plus grande sensibilité des fibres élastiques à l'action de l'élastase (Stadler R & Orfanos CE Arch. Dermatol. Res. 1978 262 (1) 97-111).

Il est connu dans l'état de la technique d'apporter, dans le tissu cutané, des molécules capables de ralentir l'activité de dégradation des fibres élastiques des espaces intercellulaires.

La présente invention a pour but de proposer une solution à ces différents problèmes, et notamment de proposer de nouvelles compositions susceptibles d'être utilisées en cosmétique ou en pharmaceutique pour limiter le vieillissement de la peau, qu'il soit chronobiologique ou photo-induit, et notamment le vieillissement généré par une diminution de l'élasticité de la peau et/ou par une dégradation du collagène dans la structure des tissus.

Il a été montré selon l'invention que les problèmes liés aux signes cutanés du vieillissement et/ou du photovieillissement associés à l'absence de contrôle de l'excès de bactéries et/ou de levures résidentes et/ou pathogènes peuvent être résolus, ou à tout le moins significativement améliorés grâce à l'association entre un composé inhibiteur de l'élastase de la famille des N-Acylaminoamides et au moins un agent antifongique ou au moins un agent antibactérien.

En conséquence, l'invention a pour objet une composition cosmétique ou dermatologique caractérisée en ce qu'elle comprend une association entre un composé inhibiteur de l'élastase de la famille des N-Acylaminoamides et au moins un agent antifongique ou au moins un agent antibactérien.

Un autre objet de l'invention consiste en un procédé de traitement cosmétique de la peau du corps ou du visage, y compris le cuir chevelu, dans lequel on applique sur la peau une composition cosmétique telle que définie ci-dessus.

Il a en effet été constaté que les composés de formule (1) présentaient une activité inhibitrice de l'activité des élastases, et qu'ils pouvaient donc être employés pour limiter et/ou combattre la dégradation des fibres élastiques.
Il s'en suit qu'ils peuvent être employés dans ou pour la préparation d'une composition, les composés ou la composition étant destinés à traiter, de manière préventive et/ou curative, les signes cutanés du vieillissement.

L'association nouvelle des composés N-Acylaminoamides avec au moins un agent antimicrobien ou au moins un agent antifongique permet de renforcer significativement l'effet antivieillissement du tissu matriciel, par l'apport d'un effet antimicrobien et antifongique. Ainsi, par une action spécifique sur l'excès de microflore cutanée, les compositions selon l'invention diminuent notamment les processus micro-inflammatoires associés à cette altération de microflore qui peuvent concourir notamment au vieillissement prématuré de la fonction épidermique.
Selon l'invention, l'élément régulateur de l'activité élastasique (i.e le dérivé N-Acylaminoamide inhibiteur de l'activité enzymatique de l' élastase leucocytaire, l'acide {2-[acétyl-(3-trifluorométhyl-phényl)-aminoj-3-méthylbutyrylamino} acétique) est combiné à un ou plusieurs actifs capables de réguler la prolifération des micro-organismes cutanés.

La composition obtenue est destinée à traiter les désordres du vieillissement et/ou à être plus spécifiquement destinée à traiter tous les désordres cutanés associés à une trop grande prolifération des bactéries et/ou des levures (P. Ovale, P. Acnes, A. Aureus) cutanées.

Préférentiellement, cette nouvelle association est utilisée dans des préparations cosmétiques de soin et/ou d'hygiène des zones exposées au soleil (scalp, corps, visage, lèvres), dans les préparation cosmétiques de soin et/ou d'hygiène des zones ulcérées, dans les dentifrices ou lotions de rinçage buccales, dans les préparations cosmétiques destinées au soin des muqueuses et, de façon générale, dans toutes les préparations cosmétiques dites " anti-vieillissement cutané " qui ont pour objectif de ralentir la destructuration chronobiologique des tissus de soutien et de l'architecture des éléments matriciels cutanés. Plus particulièrement, cette association sera réservée aux peaux comédogènes et ou acnéiques. Sans vouloir être lié par une quelconque théorie, le demandeur considère que le fait d'apporter, au niveau des kératinocytes des couches superficielles de la peau, des composés susceptibles de ralentir l'activité de dégradation des fibres élastiques des espaces intercellulaires, peut permettre de diminuer ce phénomène de vieillissement accéléré de la peau, dû à des stress cutanés superficiels et que l'association de ces composés avec un agent antimicrobien ou un agent antifongique renforce considérablement leurs effets.

Le document DE 2 322 232 décrit des compositions contenant des composés N-acylamino-amides spécifiques, actives contre les pellicules, soignant la peau, et hydratantes, entre autres.

Le document US-A-5 234 909 décrit aussi des compositions, contenant des amides dipeptidiques dérivés de glycyl-sérine, pour le traitement et le soin de la peau.

### Composés N-acylamino-amides

Les composés employés dans la présente invention répondent donc à la formule (1) suivante : 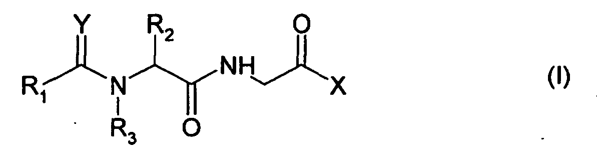 dans laquelle :
- le radical Y représente O,
- le radical R1 représente un radical méthyle, éthyle, propyle ou isopropyle, éventuellement substitué par un groupement -OH ou -P(O)-(OR)₂ avec R représentant méthyle, éthyle, propyle ou isopropyle,
- le radical R2 représente un radical méthyle, éthyle, propyle, isopropyle, n-butyle, ter-butyle ou isobutyle,
- le radical R3 représente un groupement choisi parmi l'une des formules suivantes : 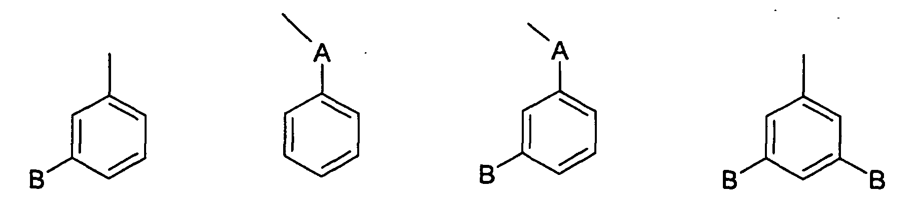 dans lesquelles
   le radical divalent A est un méthylène, un éthylène, un propylène,
   le radical B est un radical méthyle, éthyle, propyle ou isopropyle, substitué par un ou plusieurs halogènes, notamment chlore, brome, iode ou fluor, et préférentiellement totalement halogéné (perhalogéné), tel que perfluoré; on peut en particulier citer le radical perfluorométhyle (-CF3) comme tout particulièrement préféré,
- le radical X représente un radical choisi parmi -OH, - OCH₃, -OC₂H₅, -O-C₃H₇ ou -OC₄H₉,
ses sels d'acide minéral ou organique, ses isomères optiques, sous forme isolée ou en mélange racémique.

Parmi les composés particulièrement préférés, on peut citer :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

Les composés selon l'invention peuvent être aisément préparés par l'homme du métier sur base de ses connaissances générales. On peut notamment faire réagir ensemble un acide carboxylique, un aldéhyde, un composé aminé et un isonitrile, selon la réaction de Ugi.

Bien entendu, lors de la synthèse des composés selon l'invention, et en fonction de la nature des différents radicaux présents sur les composés de départ, l'homme du métier pourra veiller à protéger certains substituants pour que ceux-ci n'interviennent pas dans la suite des réactions.

La quantité de composé à utiliser dans les compositions selon l'invention peut être aisément déterminée par l'homme du métier, en fonction de la nature du composé utilisé, de la personne à traiter et/ou de l'effet recherché. D'une manière générale, cette quantité peut être comprise entre 0,00001 et 20% en poids par rapport au poids total de la composition, préférentiellement entre 0,001 et 10% en poids.
Les composés de formule (I) peuvent notamment être employés, seul ou en mélange, dans une composition qui comprend un milieu physiologiquement acceptable, notamment dans une composition cosmétique ou pharmaceutique qui comprend donc par ailleurs un milieu cosmétiquement ou pharmaceutiquement acceptable.
Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.
D'une manière générale, ce milieu peut être anhydre ou aqueux: Il peut ainsi comprendre une phase aqueuse et/ou une phase grasse.

### Agents antifongiques préférés

Selon l'invention, on entend par agent antifongique, toute substance capable d'inhiber ou d'empêcher la croissance des levures en particulier celles que l'on trouve à la surface de l'épiderme riche en glandes sébacées et notamment à la surface du cuir chevelu comme par exemple le *Pityrosporum ovale* et ses variétés (*Pityrosporum orbiculare* et *Malassezia furfur*)

Parmi les agents antifongiques employés selon l'invention on peut plus particulièrement citer la terbinafine, le zinc pirythione, le sulfure de sélénium, les goudrons et leurs dérivés, l'acide undécylénique et ses sels, les dérivés d'hydroxypyridone tels que le CICLOPIROX : 6-cyclohexyl 1-hydroxy 4-méthyl 2-(1 H)-pyridone ou l'OCTOPIROX : 1-hydroxy 4-méthyl 6-(2,4,4,-triméthylpentyl)-2-(1H)-pyridone, des agents iminudazolés tels que ketoconazole, flutrinazole, lanoconazole, neticonazole, sertaconazole nitrate, omoconazole nitrate, fenticonazole nitrate, croconazole, butoconazole nitrate, sulconazole nitrate, bifonazole, oxiconazole nitrate, troconazole, des agents triazolés tels que terconazole, fluconazole et itraconazole, la terbinafine, la butenafine..

Ces agents antifongiques sont de préférence présents dans les compositions conformes à l'invention à une concentration pouvant varier entre 0,0001 et 10 % en poids environ par rapport au poids total de la composition. Encore plus préférentiellement, la concentration en agents antifongiques peut varier entre 0,01 et 2 % en poids par rapport au poids total de la composition.

### Agents antibactériens préférés

Une première famille de composés antibactériens est constituée du miel et de ses dérivés, dont il est montré qu'ils modifient l'attachement des microorganismes sur les cellules, en particulier sur les cellules de la peau et/ou les muqueuses.

On entend par miel le produit de transformation par les abeilles du nectar et/ou du miellat des fleurs.

Bien que la teneur de ses différents constituants puisse varier selon sa provenance, le miel est généralement au moins un mélange de glucose, de lévulose, de maltose, de saccharose, ainsi que d'autres composants comme des protéines, des acides organiques, des lactoses, des substances minérales, des oligo-éléments, des vitamines, de nombreux enzymes des substances aromatiques et bien évidemment de l'eau.

Le miel peut être un miel non fermenté ne contenant pas d'hydroxyacides ou un miel fermenté contenant des hydroxyacides.

Le miel peut être de toute provenance. Il peut s'agir notamment de miel de fleurs d'acacias, de tilleul, de lavande, de châtaignier, de résineux, de fleurs d'oranger, de toutes fleurs de montagne, du "Gatinais".

Préférentiellement, on utilise selon l'invention du miel de fleurs d'acacias.

Par miel de fleurs d'acacias on entend tout miel obtenu à partir du nectar et/ou du miellat de fleurs de Robinier (*Robinia pseudacacia L.*).

La quantité de miel utilisable selon l'invention dépend bien évidemment de l'effet recherché et doit être en une quantité efficace pour empêcher partiellement, voire totalement, l'adhésion des micro-organismes ou pour faciliter le détachement des micro-organismes.

Une seconde famille de composés antibactériens préférés selon l'invention consistent en des composés de la famille des hydroxystilbènes.

On utilisera de préférence les hydroxystilbènes répondant à la formule générale (IV) ci-dessous : 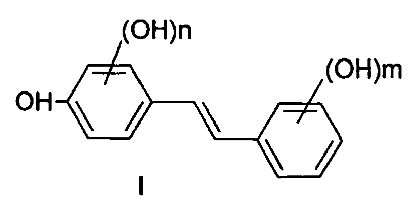 dans laquelle n est un nombre entier compris entre 1 et 4 inclusivement et m est un nombre entier compris entre 1 et 5 inclusivement. Ces composés peuvent être sous une forme Cis ou Trans.
Selon l'invention, le terme hydroxystilbène recouvre aussi bien les composés de formule I que leurs dérivés hydroxyalkylés.

Les hydroxystilbènes sont des composés que l'on retrouve à l'état naturel dans les végétaux de la classe des spermatophytes et particulièrement dans la vigne. On trouve de tels composés comme par exemple le resvératrol dans le raisin et dans le vin.
Selon l'invention les hydroxystilbènes peuvent être utilisés seuls ou en mélanges de toute nature et peuvent être d'origine naturelle ou synthétique.

Les hydroxystilbènes utilisables selon l'invention sont préférentiellement choisis parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène, (ou resveratrol)
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2, 3',4,4',6-pentahydroxystilbène,
le 2,2',4,4',6,6'-hexahydroxystilbène.

De manière tout à fait préférée, on utilise selon l'invention du 3,4',5-trihydroxystilbène (ou resvératrol).

Une troisième famille de composés antibactériens utilisables selon l'invention sont les composés antibactériens halogénés.

Selon l'invention, on entend par agent antibactérien halogéné, toute substance comportant au moins un atome d'halogène et capable d'inhiber ou d'empêcher la croissance de la flore bactérienne présente à la surface de l'épiderme riche en glandes sébacées.

Parmi les agents antibactériens halogénés employés selon l'invention, on peut plus particulièrement citer les agents antibactériens chlorés tels que le Triclosan qui est le 5-chloro-2-(2,4-dichlorophénoxy)phénol, vendu sous la dénomination commerciale IRGASAN par la Société CIBA-GEIGY, la chlorhexidine et ses dérivés, et le chloramphénicol.
Parmi les autres agents anti-bactériens utilisés préférentiellement selon l'invention, on peut citer Ides sucres complexes et notamment le syallyllactose capable de limiter particulièrement la multiplication d'Hélicobacter Pylori. Par analogie, des combinaisons d'antibiotiques ayant démontré plus particulièrement une très bonne efficacité pour enrayer H. Pylori dans d'autres pathologies. Comme les combinaisons (omeprazole/amoxicillin) ou agent pharmaceutiques comme le metrodinazole, la clarithromycine sont décrits par Miehjlke et al dans Digestion (1988) 59(6) :646-50.

Les agents antibactériens sont présents de préférence à une concentration comprise entre 0,001 % et 10 % en poids total de la composition, de préférence entre 0,01% et 2%.

De préférence, le rapport pondéral de l'agent antifongique à l'agent antibactérien varie de 0,2 à 10.

L'association d'au moins un composé N-acylamino-amide et d'au moins un agent antibactérien et/ou d'au moins un agent antifongique peut notamment être employée, seule ou en mélange, dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.

Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.
Ladite phase aqueuse peut comprendre en outre des alcools tels que des monoalcools en C₁-C₆ et/ou des polyols tels que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux; un shampooing antiparasitaire;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

Les compositions selon l'invention trouvent une application préférée comme composition de soin de la peau du visage, de type anti-rides ou anti-age, et comme composition de protection solaire ou après-soleil.

La présente invention a également pour objet un procédé de traitement cosmétique de la peau du corps ou du visage, y compris le cuir chevelu, dans lequel on applique sur la peau une composition cosmétique comprenant une association entre un composé de la famille des N-acylamino-amides et au moins un agent antibactérien ou au moins un agent antifongique, à laisser celle-ci en contact puis éventuellement à rincer.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs; application d'une lotion pour cuir chevelu sur cheveux mouillés; application de dentifrice sur les gencives.

L'invention est illustrée plus en détail dans les exemples suivants.

### Exemple 1

### Préparation du {2-[acétyl-(3-trifluorométhyl-phényl)-amino]-3-méthyl-butyrylamino} acétate d'éthyle de formule :

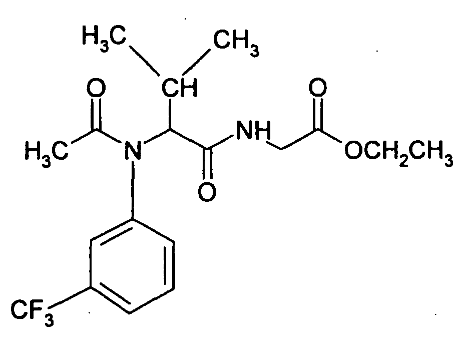

On mélange 0,63 ml d'isobutyraldéhyde et 1 ml de trifluorométhylamine (1,15 eq) dans 15 ml de méthanol, sous agitation. On laisse réagir 15 minutes à 20°C, puis on ajoute 0,46 ml d'acide acétique (1,15 eq) et on laisse réagir 10 minutes à 20°C. On ajoute alors 0,8 ml d'isocyanoacétate d'éthyle à 95% (1 eq) et on laisse réagir 48 heures à 20°C.

On concentre le milieu réactionnel au rotovapor et on purifie le résidu sur colonne de silice (éluant : heptane : 3 / acétate d'éthyle : 7; Rf = 0,5).

On obtient 2,45 g de composé sous forme de solide cireux, d'où un rendement de 91 %.

RMN ¹H (200 MHz; CDCl3) δ ppm : 0,9 (6H;q), 1,3 (3H;t), 1,8 (3H;s), 2,3 (1H; m), 4,0 (2H,q), 4,2 (2H;q), 4,4 (2H;d), 7,3 (1 H;t), 7,5 (4H;m)

### Exemple 2

### Préparation de l'acide {2-[acétyl-(3-trifluorométhyl-phényl)-amino]-3-méthyl-butyrylamino} acétique de formule :

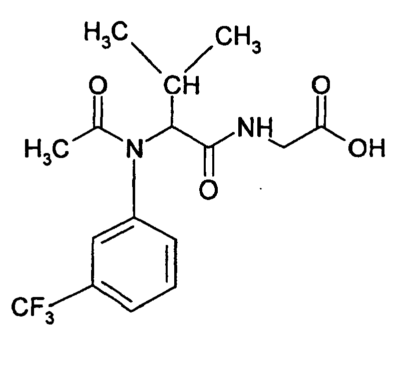

On solubilise 2 g de composé préparé à l'exemple 1 dans 30 ml d'acétone. On ajoute 30 ml de soude 2N et on laisse réagir pendant 6 heures à 20°C.

On concentre le milieu réactionnel au rotovapor. On acidifie la phase aqueuse résiduelle à pH 2 par ajout d'HCl concentré puis on extrait par CH2Cl2.

La phase organique est concentrée à sec après séchage sur sulfate de sodium.

On obtient un résidu qui est solubilisé par un mélange eau basique à 10% d'éthanol, puis de nouveau acidifié par HCl concentré à pH 2. On extrait une nouvelle fois par Ch2Cl2 et on sèche la phase organique sur sulfate de sodium, on la filtre et on la concentre à sec sous vide au rotovapor.

On obtient 1,3 g de composé sous forme d'un solide légèrement marron clair, d'où un rendement de 70%.

RMN ¹H (200 MHz; DMSO) δ ppm : 0,9 (6H;q), 3,7 (2H;m), 1,8 (4H;m), 4,8 (2H;d), 7,6 (4H,m),8,4 (1H;t), 12,5 (1H;s)

### Exemple 3

On a déterminé in vitro l'activité anti-élastasique de composés selon l'invention, vis-à-vis de l'élastase leucocytaire humaine (ELH).

### Le test est effectué de la manière suivante :

On laisse incuber, à 37°C, pendant 60 minutes, un substrat Me-OSAAPV-p-NA (Méthyl-O-succinate alanine alanine proline valine-p-nitroanilide) sur lequel est appliqué de l'ELH (40 milli-unités par ml) et 0,1% du composé à tester.
On détermine ensuite par spectrophotométrie le % d'inhibition de l'activité élastase témoin.

Les composés testés sont les suivants :
Composé A : acide {2-[acétyl-(3-trifluorométhyl-phényl)-amino]-3-méthyl-butyryl-amino} acétique
Composé B : (2-{benzyl-[(diethoxy-phosphoryl)-acétyl]-amino}-3-méthyl-butyryl-amino) acétate d'éthyle
Composé C : acide [2-(acétyl-benzyl-amino)-3-méthyl-butyrylamino] acétique
Composé D : [2-(acétyl-benzyl-amino)-3-méthyl-butyrylamino] acétate d'éthyle

On obtient les résultats suivants :

| Composé (concentration : 0,1 %) | % d'inhibition de l'activité élastase témoin |
|---|---|
| Composé A | 67% |
| Composé B | 17% |
| Composé C | 20% |
| Composé D | 13% |

On détermine de la même manière le % d'inhibition de l'activité élastase témoin pour le composé A, à différentes concentrations.

On obtient les résultas suivants :

| Concentration du composé A | % d'inhibition de l'activité élastase témoin |
|---|---|
| 0,01 % | 53% |
| 0,05% | 50% |
| 0,1% | 68% |
| 0,2% | 68% |

Le composé A provoque donc une forte inhibition de l'activité élastase, même en une quantité faible.

### Exemple 4

On a évalué l'activité ex vivo du composé de l'exemple 2 sur des peaux humaines en survie traitées par de l'élastase leucocytaire humaine (ELH).

### Le test est effectué de la manière suivante :

Des coupes fraîches de peaux humaines, provenant de 2 donneurs différents, sont traitées pendant 2 heures, à 20°C, par 20 µl de solution tampon (pH 7,4) comprenant éventuellement 10 µg/ml d'ELH et éventuellement 0,1% du composé à tester, éventuellement préalablement mis en solution dans l'éthanol.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques | |
|---|---|---|
| | Peau 1 | Peau 2 |
| Témoin (peau non traitée) | 12,7% | 15,25% |
| Peau traitée avec ELH | 4,85% | 6,85% |
| Peau traitée avec ELH + composé de l'exemple 2 | 13,95% | 11,85% |

On constate donc que le composé selon l'invention génère une protection significative des peaux vis-à-vis de la destruction des fibres élastiques induite par l'élastase.

### Exemple 5

On a évalué l'activité ex vivo du composé de l'exemple 2 sur des peaux humaines en survie traitées par de l'élastase leucocytaire humaine (ELH).

### Le test est effectué de la manière suivante :

Des fragments de peau humaine normale provenant de trois donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).
Le milieu de culture est renouvelé tous les trois jours.

On ajoute sur les fragments de peau éventuellement 0,5 µg d'ELH par ml de milieu de culture.

On ajoute également, tous les deux jours, 5 µl du composé à tester, préalablement mis en solution à 0,2% en poids dans l'éthanol.

Les peaux sont maintenues en survie pendant 10 jours à 37°C.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques |
|---|---|
| Témoin (peau non traitée) | 7,4% |
| Peau traitée avec ELH | 5,1% |
| Peau traitée avec ELH + composé de l'exemple 2 | 7,1 % |

On constate donc que le composé selon l'invention génère une protection significative des peaux vis-à-vis de la destruction des fibres élastiques induite par l'élastase.

### Exemple 6

On a évalué l'activité du composé de l'exemple 2 sur des peaux humaines en survie irradiées par des UVA (8 J/cm²).

### Le test est effectué de la manière suivante :

Des fragments de peau humaine normale provenant de quatre donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores 12 µm).

Le milieu de culture est renouvelé tous les trois jours.

On ajoute sur les fragments de peau, tous les deux jours, 5 µl du composé à tester, préalablement mis en solution à 0,2% dans l'éthanol.

Les peaux sont maintenues en survie pendant 7 jours à 37°C.

Les peaux sont irradiées une seule fois à 8 J/cm² (lampe Vilbert-Lourmat RMX-3W).

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | | |
|---|---|---|
| | | |

| | Analyse morphométrique des fibres élastiques (derme superficiel) | Analyse morphométrique du collagène (derme superficiel). |
|---|---|---|
| | | |
| Peau non traitée | 6,75% | 87%. |
| | | |
| | | |
| Peau traitée par des UVA (8 J/cm²) | 3,9% | 81%. |
| | | |
| Peau traitée par des UVA (8 J/cm²) + composé de l'exemple 2 | 6,8% | 92%. |

On constate que le composé selon l'invention a bien une activité vis-à-vis de la dégradation des fibres élastiques dans le derme superficiel de peaux irradiées par des UVA.

### Exemple 7

On a comparé l'activité de différents miels dans le modèle de détachement cellulaire *in vitro* chez l'homme.

Ce test *in vitro* de criblage d'un agent actif sur l'adhésion cellulaire est réalisé sur kératinocytes humains différenciés. Le principe du test repose sur le fait que l'inhibition de l'adhésion cellulaire induit la libération de kératinocytes humains différenciés. (Les résultats de ce test sont assimilables à l'adhésion des micro-organismes à des cornéocytes - confirmés dans l'exemple 8).

Le pouvoir de détachement cellulaire du produit testé va être d'autant plus grand que le nombre de kératinocytes différenciés libérés sera important. Le protocole du test est le suivant : à partir de biopsies de peau humaine, après séparation de l'épiderme du derme, les kératinocytes sont dissociés par action enzymatique à la trypsine et mis en culture à la concentration de 2.10⁵ cellules/ml selon les techniques de culture cellulaires traditionnelles connues de l'homme du métier.

La croissance et la différenciation des kératinocytes est obtenue par culture durant 10 à 20 jours

Après élimination du milieu de culture, l'activité du produit à tester est évaluée. Deux prélèvements de milieu de culture sont réalisés à T0 et T60, c'est à dire avant l'ajout du produit à tester (T0) et 60 minutes après cet ajout (T60). Les prélèvements ainsi effectués sont analysés au cytomètre de flux pour dénombrer la population de cornéocytes présents dans le milieu.

Le cytomètre de flux permet de distinguer les populations de cornéocytes et de kératinocytes par traitement à l'acridine orange spécifique de l'acide désoxyribonucléique (ADN) des cellules. Cette coloration est spécifique des kératinocytes puisque les cornéocytes ne possèdent pas de noyaux donc pas d'ADN.

Les résultats de ces études sont résumés dans le tableau suivant.

| | Témoin | A | B : 0.1 % | C : 0.1 % | D : 0.1 % | E : 0.1 % |
|---|---|---|---|---|---|---|
| I | 1958 | 4111 | 2111 | 3055 | 3111 | 4611 |
| II | 0 | 110 | 7.8 | 56 | 58.9 | 135.5 |

| | | | | | | |
|---|---|---|---|---|---|---|
| Témoin : Milieu de culture sans composé : Témoin négatif. A : Acide 2 hydroxy, 5 octanoylbenzoïque à la concentration de 5.10⁻⁵ M : (Témoin positif). B : Miel de lavande C : Miel de châtaignier D : Miel de résineux E : Miel d'acacias I : Nombre de cornéocytes détachés (mesure à T60 diminuée de la mesure à T0) II : Activité des miels en % par rapport au témoin. | | | | | | |

Les résultats confirment l'activité inhibitrice du miel sur l'adhésion cellulaire. La provenance de celui-ci semble importante et le miel d'acacias apparaît comme le meilleur inhibiteur de l'adhésion cellulaire

### Exemple 8

On a procédé à une mesure de l'activité du miel sur l'adhésion de microorganismes sur la peau ou sur les muqueuses humaines.

Le miel d'acacias, le plus actif dans le test précédent, a été retenu pour le test sur un modèle d'adhésion bactéries/peau, tel que décrit ci-après.

Ce modèle d'adhésion bactéries/peau *ex vivo* est basé sur l'utilisation de peau humaine intacte montée en sandwich entre une plaque 96 puits sans fond et un support Plexiglas. Les puits individualisés étanches permettent de tester dans un format standard l'effet du miel d'acacias sur l'adhésion de *Staphylococcus epidermis* (bactéries résidentes) à la surface de la peau. Les bactéries sont radio marquées en phase de croissance par incorporation de 3H-thymidine. Cette flore bactérienne est ensuite déposée dans chaque puits. Après une heure d'incubation à 20°C, les puits sont lavés trois fois avec du tampon phosphate salin (PBS) ce qui permet d'éliminer les bactéries libres. Le miel d'acacias est ensuite ajouté à 5 et 10% et incubé 1 heure à 20°C. A l'issue de cette incubation le contenu de chaque puits est récupéré et compté en scintillation liquide (bactéries désorbées). Les puits sont ensuite lavés avec une solution chaotropique qui permet d'éluer les bactéries adhérentes. Les solutions de lavages sont également compté en scintillation liquide (bactéries adhérentes). Les témoins positifs et négatifs (respectivement Fucogel® Acide (poly-[(α-1,3)-Fuc-(α-1,3)-Gal-(α-1,3)-galacturonique]) et Bioecolia® (Oligo-(α-1,4)-Gluc-(α-1,2)-Fruc-(α-1,6)-Gluc]) décrits dans la publication de Wolf F., et al. (Edition du 19^{ième} Congres IFSCC, Sydney, 1996), sont inclus à l'étude comme références.

| | | | | | |
|---|---|---|---|---|---|
| | | | | | |

| | Témoin | Bioécolia® (10%) | Fucogel® (10%) | miel d'acacias (5%) | miel d'acacias (10%) |
|---|---|---|---|---|---|
| Bactéries désorbées | 100% | 89% (p>0.05) | 257% (p<0.01) | 200% (p<0.01) | 228% (p<0.01) |
| Bactéries adhérentes | 100% | 76% (p>0.05) | 12% (p<0.01) | 59% (p<0.01) | 30% (p<0.01) |

| | | | | | |
|---|---|---|---|---|---|
| Témoin : tampon PBS seul. p : analyse de variance - test de comparaison multiple de Dunnett. | | | | | |

Le fucogel® (contrôle positif) stimule la désorption des bactéries et inhibe l'adhésion. Bioécolia® (contrôle négatif) ne modifie pas l'adhésion de façon significative. Le miel d'acacias à 5 et 10% stimule la désorption des bactéries (228 et 200%) et inhibe l'adhésion (30 et 59%).

En conclusion, les résultats présentés ci-dessus confirment bien le rôle du miel d'acacias dans la régulation de l'adhésion des bactéries sur la peau humaine.

Cette activité est dose dépendante et identique à la celle de la référence positive.

### Exemple 9

On a mesuré l'activité du resvératrol sur l'adhésion de micro-organismes sur la peau ou sur les muqueuses humaines.

L'activité du resvératrol (3,4',5 trihydroxystilbène) sur l'adhésion de micro-organismes sur la peau ou sur les muqueuses humaines est testée sur un modèle d'adhésion bactéries/peau, tel que décrit ci-après.

Ce modèle d'adhésion bactéries/peau *ex vivo* est basé sur l'utilisation d'explants de peau humaine intacte montée en sandwich entre une plaque 96 puits sans fond et un support en Plexiglas. Les puits individualisés étanches permettent de tester dans un format standard l'effet du resvératrol sur l'adhésion de *Staphylococcus epidermis* (bactéries résidentes) à la surface de la peau. Les bactéries sont radio marquées en phase de croissance par incorporation de 3H-thymidine. Cette flore bactérienne est ensuite déposée dans chaque puits. Après une heure d'incubation à 20°C, les puits sont lavés trois fois avec du tampon phosphate salin (PBS) ce qui permet d'éliminer les bactéries libres. Le resvératrol est ensuite ajouté à 0,1 µM et 10 µM et incubé 1 heure à 20°C. A l'issue de cette incubation le contenu de chaque puits est récupéré et compté en scintillation liquide (bactéries désorbées). Les puits sont ensuite lavés avec une solution chaotropique qui permet d'éluer les bactéries adhérentes. Les solutions de lavages sont également compté en scintillation liquide (bactéries adhérentes). Un témoin positif (Fucogel® Acide (poly-[(α-1,3)-Fuc-(α-1,3)-Gal-(α-1,3)-galacturonique]) est inclus à l'étude comme référence.

| | Témoin | Fucogel® (10%) | resvératrol 0,1 µM | resvératrol 10 µM |
|---|---|---|---|---|
| Bactéries adhérentes | 100% | 42% (p<0.01) | 71% (p<0.01) | 64% (p<0.01) |

| | | | | |
|---|---|---|---|---|
| Témoin : tampon PBS seul. p : analyse de variance - test de comparaison multiple de Dunnett. | | | | |

Le Fucogel® (contrôle positif) stimule la désorption des bactéries et inhibe l'adhésion. Le resvératrol à 0,1 µM et 10 µM inhibe l'adhésion des bactéries (29 et 36% d'inhibition respectivement).

En conclusion, les résultats présentés ci-dessus confirment bien le rôle du resvératrol dans la régulation de l'adhésion des bactéries sur la peau humaine.

Cette activité est dose dépendante.

### Exemple 10 : composition pour application topique

On prépare l'émulsion suivante de façon classique (% en poids) :
- composé de l'Exemple 1 (acide de l'exemple 2) 1 %
- isostéarate de propylène glycol 13 %
- polyéthylène glycol (8 OE) 5 %
- propylène glycol 3 %
- pentylène glycol 3 %
- stéarate de glycéryle et stéarate de polyéthylène glycol (100 OE) 5 %
- mono-stéarate de sorbitane oxyéthyléné (20 OE) 0,5 %
- alcool cétylique oxyéthyléné (20 OE) oxypropyléné (5 OP) 1 %
- gélifiants 0,5 %
- benzoates d'alkyle en C₁₂₋₁₅ 4 %
- éthanol 3 %
- hydroxyde de sodium 0,12 %
- conservateurs qs
- eau qsp 100 %
- 1-hydroxy-4-methyl 6-(2,4,4-trimethylpentyl)-2(1H) pyridone- (Octopirox) 0,25 %
- Miel d'acacia (*Robinia pseudacacia L*.) 0,5 %

### Exemple 11: crème de soin du visage

On prépare l'émulsion huile-dans-eau suivante de façon classique (% en poids) :
- Miel d'acacia (*Robinia pseudacacia L.*) 0,5 %
- Composé de l'exemple 2 1 %
- Stearate de glycérol 2%
- Polysorbate 60 (Tween 60® vendu par la société ICI) 1 %
- Acide stéarique 1,4%
- Triéthanolamine 0,7%
- Carbomer 0,4 %
- Fraction liquide du beurre de karité 12%
- Perhydrosqualène 12%
- Antioxydant qs
- Parfum qs
- Conservateur qs
- Eau qsp 100 %

### Exemple 12: Composition dermatologique pour application topique

On prépare le lait suivant de façon classique (% en poids) :
- Huile de vaseline 7 %
- Composé de l'exemple 2 1%
- Monostéarate de glycéryle, stéarate de polyéthylène glycol (100 OE) 3 %
- Polymère carboxyvinylique 0,4 %
- Alcool stéarylique 0,7 %
- Protéines de soja 3 %
- NaOH 0.4 %
- Conservateur qs
- Eau qsp 100 %
- Ketoconazole 0,5 %
- 5-chloro-2-(2,4-dichlorophenoxyl)phenol- (Triclosan) 1 %

### Exemple 13 : lotion capillaire

On prépare la lotion suivante de façon classique (% en poids) :
- -1-hydroxy 4 methyl-6-(2,4,4-trimethylpentyl)-2-(1H)pyridone- (Octopirox) 0,25 %
- composé de l'Exemple 2 1 %
- propylène glycol 23 %
- éthanol 55 %
- eau qsp 100 %

On peut appliquer cette lotion sur le scalp des individus alopéciques, pour prévenir les effets des UV, avant et/ou après exposition au soleil.

### Exemple 14 : lotion antichute

On prépare la lotion suivante de façon classique (% en poids) :
- composé de l'Exemple 2 1 %
- propylène glycol 23 %
- éthanol 55 %
- Aminexil 1,5 %
- eau qsp 100 %
- 1-hydroxy 4 methyl-6-(2,4,4-trimethylpentyl)-2-(1 H)pyridone- (octopirox) 0,25 %
- 5-chloro-2-(2,4-dichlorophenoxyl)phenol- (Triclosan) 0,5 %
- 2,6 diaminopyridinine N-oxyde- (Aminexil) 2 %

On peut appliquer cette lotion antichute sur le scalp des individus alopéciques.

## Revendications

1. Composition cosmétique ou dermatologique comprenant une association entre un composé inhibiteur de l'élastase de la famille des N-Acylaminoamides et au moins un agent antifongique ou au moins un agent antibactérien,
**caractérisée en ce que** le composé inhibiteur de la famille des N-Acylaminoamides est un composé de formule (1) : 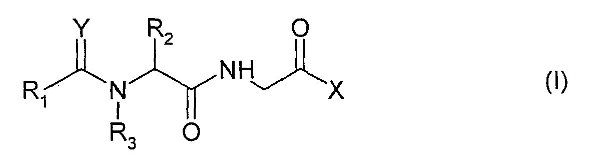 dans laquelle :
- Y représente l'oxygène
- le radical R1 représente un radical méthyle, éthyle, propyle ou isopropyle, éventuellement substitué par un groupement -OH ou -P(O)-(OR)₂ avec R représentant méthyle, éthyle, propyle ou isopropyle; et/ou
- le radical R2 représente un radical méthyle, éthyle, propyle, isopropyle, n-butyle, ter-butyle ou isobutyle; et/ou
- le radical R3 représente un groupement choisi parmi l'une des formules suivantes : 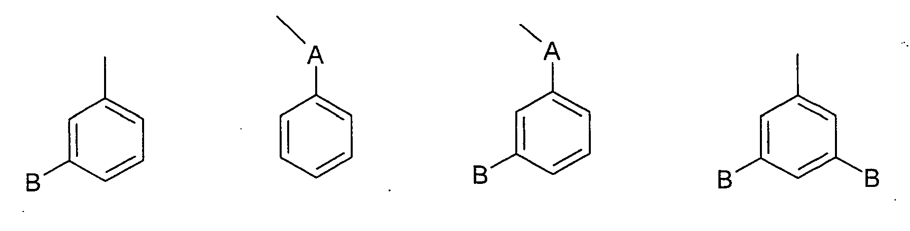 dans lesquelles le radical divalent A est un méthylène, un éthylène, un propylène et/ou le radical B est un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF3).
- le radical X représente un radical choisi parmi -OH, -OCH₃, -OC₂H₅, -OC₃H₇ ou -OC₄H₉,
ses sels d'acide minéral ou organique, ses isomères optiques, sous formes isolée ou en mélange racémique.

2. Composition selon la revendication 1, dans laquelle le composé de formule (1) est choisi parmi les composés suivants :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

3. Composition selon l'une des revendications précédentes, dans laquelle le composé inhibiteur de l'élastase de la famille des N-Acylaminoamides est présent en une quantité comprise entre 0,00001 et 20% en poids par rapport au poids total de la composition, préférentiellement entre 0,001 et 10% en poids.

4. Composition selon l'une des revendications précédentes, **caractérisée en ce que** l'agent antifongique est choisi parmi les composés suivants : les composés imidazoles et leurs dérivés ; a terbinafine, le zinc pirythione, le sulfure de sélénium, les goudrons et leurs dérivés, l'acide undécylénique et ses sels, les dérivés d'hydroxypyridone tels que le CICLOPIROX : 6-cyclohexyl 1-hydroxy 4-méthyl 2-(1 H)-pyridone ou l'OCTOPIROX : 1-hydroxy 4-méthyl 6-(2,4,4,-triméthylpentyl)-2-(1 H)-pyridone.

5. Composition selon l'une des revendications précédentes, **caractérisée en ce que** les agents antifongiques sont présents à une concentration comprise entre 0,0001 % et 10% en poids total de la composition, préférentiellement entre 0,01 et 2 % en poids par rapport au poids total de la composition

6. Composition selon l'une des revendications précédentes, **caractérisée en ce que** les agents antibactériens sont choisis parmi les composés suivants : le miel, un composé de la famille des hydroxystilbènes et un agent antibactérien halogéné et/ou un sucre complexe comme le syallyllactose ou des agents antibiotiques classiques.

7. Composition selon l'une des revendications précédentes, **caractérisée en ce que** les agents antibactériens sont présents à une concentration comprise entre 0,001 % et 10 % en poids total de la composition, de préférence entre 0,01 % et 2%.

8. Composition selon l'une des revendications précédentes, **caractérisée en ce qu'**elle comprend au moins un agent antifongique et au moins un agent antibactérien.

9. Composition selon la revendication 7, **caractérisée en ce que** le rapport pondéral de l'agent antifongique à l'agent antibactérien varie de 0,2 à 10.

10. Composition selon l'une des revendications précédentes, se présentant sous la forme d'une composition cosmétique ou dermatologique destinée au soin et/ou au traitement des zones ulcérées ou ayant subi un stress ou microstress cutané, notamment généré par une exposition aux UV et/ou la mise en contact avec un produit irritant.

11. Composition selon l'une des revendications 1 à 9, se présentant sous la forme :
- d'un produit de soin, de traitement, de nettoyage ou de protection, de la peau du visage ou du corps y compris le cuir chevelu, et des muqueuses tel qu'une composition de soin (de jour, de nuit, hydratante) du visage ou du corps; une composition anti-rides ou anti-age pour le visage; une composition matifiante pour le visage; une composition pour les peaux irritées;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

12. Composition selon l'une des revendications 1 à 9, se présentant sous la forme d'une composition de soin de la peau du visage, de type anti-rides ou anti-age, ou d'une composition de protection solaire ou après-soleil.

13. Procédé de traitement cosmétique de la peau du corps ou du visage, y compris le cuir chevelu, dans lequel on applique sur la peau ou les muqueuses une composition cosmétique telle, que définie dans l'une des revendications 1 à 12.

## Claims

1. Cosmetic or dermatological composition comprising a combination of an inhibitor of elastase of the N-acylaminoamides and at least one antifungal agent or at least one antibacterial agent **characterized in that** the inhibitor of the N-acylaminoamides is a compound of formula (1): 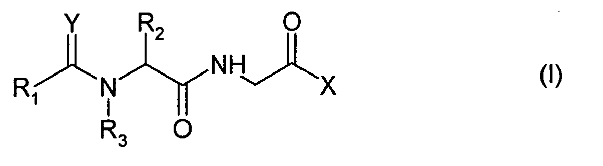 wherein:
- Y represents oxygen,
- the radical R1 represents a methyl, ethyl, propyl or isopropyl radical, optionally substituted by an -OH or -P(O)-(OR)₂ group with R representing a methyl, ethyl, propyl or isopropyl radical; and/or
- the radical R2 represents a methyl, ethyl, propyl, isopropyl, n-butyl, tert-butyl or isobutyl radical; and/or
- the radical R3 represents a group selected from one of the following formulae: 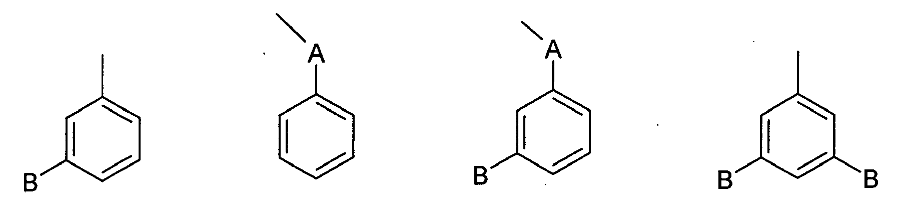 in which the divalent radical A is a methylene, ethylene, propylene radical and/or the radical B is a methyl, ethyl, propyl or isopropyl radical substituted by one or more halogens, in particular chlorine, bromine, iodine or fluorine, and preferably completely halogenated (perhalogenated), such as perfluorinated, in particular the perfluoromethyl radical (-CF3).
- the radical X represents a radical selected from -OH, -OCH₃, -OC₂H₅, - OC₃H₇ or -OC₄H₉, the mineral or organic acid salts thereof, the optical isomers thereof in isolated form or as a racemic mixture.

2. Composition according to Claim 1, wherein the compound of formula (I) is selected from the following compounds:
- {2-[acetyl-(3-trifluoromethyl-phenyl)-amino]-3-methyl-butyrylamino} acetic acid
- {2-[acetyl-(3-trifluoromethyl-phenyl)-amino]-3-methyl-butyrylamino} ethyl acetate
- [2-(acetyl-benzyl-amino)-3-methyl-butyrylamino] acetic acid
- [2-(acetyl-benzyl-amino)-3-methyl-butyrylamino] ethyl acetate
- (2-{benzyl-[(diethoxy-phosphoryl)-acetyl]-amino}-3-methyl-butyrylamino) ethyl acetate.

3. Composition according to one of the preceding Claims, wherein the inhibitor of elastase of the N-acylaminoamides is present in a quantity included between 0.00001 and 20% by weight based on the total weight of the composition, and preferably between 0.001 and 10% by weight.

4. Composition according to one of the preceding Claims, **characterized in that** the antifungal agent is selected from the following compounds: the imidazole compounds and the derivatives thereof; terbinafine, zinc pirythione, selenium sulfide, the tars and the derivatives thereof, undecylenic acid and the salts thereof, the derivatives of hydroxypyridone such as CICLOPIROX: 6-cyclohexyl 1-hydroxy 4-methyl 2-(1H)-pyridone or OCTOPIROX: 1-hydroxy 4-methyl 6-(2,4,4-trimethylpentyl)-2-(1H)-pyridone.

5. Composition according to one of the preceding Claims, **characterized in that** the antifungal agents are present at a concentration included between 0.0001% and 10% based on the total weight of the composition, and preferably between 0.01 and 2% based on the total weight of the composition.

6. Composition according to one of the preceding Claims, **characterized in that** the antibacterial agents are selected from the following compounds: honey, a compound of the hydroxystilbenes and a halogenated antibacterial agent and/or a sugar complex like syallyllactose or conventional antibiotic agents.

7. Composition according to one of the preceding Claims, **characterized in that** the antibacterial agents are present at a concentration included between 0.001 % and 10% based on the total weight of the composition, and preferably between 0.01 % and 2%.

8. Composition according to one of the preceding Claims, **characterized in that** it comprises at least one antifungal agent and at least one antibacterial agent.

9. Composition according to Claim 7, **characterized in that** the weight ratio of the antifungal agent to the antibacterial agent varies from 0.2 to 10.

10. Composition according to one of the preceding Claims in the form of a cosmetic or dermatological composition designed for the care and/or treatment of ulcerated areas or areas subjected to a cutaneous stress or microstress, in particular generated by exposure to UV and/or being placed in contact with an irritant product.

11. Composition according to one of the Claims 1 to 9, in the form of:
- a product for the care, treatment, cleansing or protection of the skin of the face or body including the scalp and mucous membranes such as a care composition (daytime, nighttime, hydrating) for the face or body; an anti-wrinkle or anti-age composition for the face; a composition rendering the face mat; a composition for irritated skin;
- a sun protection composition, an artificial tanning composition (self-tanning) or care composition after exposure to the sun;
- a composition for the hair, and in particular a sun protection cream or gel; a care composition for the scalp, in particular against hair loss or stimulating hair growth;
- a product for the make-up of the skin or the face, body or lips, such as foundation make-up, complexion cream, rouge or eyelid make-up, a free or compact powder, anti-shadow stick, a camouflaging stick, a lipstick, a lip care composition;
- a product for buccal hygiene such as toothpaste or a mouthwash.

12. Composition according to one of the Claims 1 to 9, in the form of a care composition for the skin of the face of the anti-wrinkle or anti-age type, or of a sun protection or after-sun composition.

13. Cosmetic treatment method for the skin of the body or face, including the scalp, wherein a cosmetic composition such as defined in any one of claims 1 to 12 is applied on the skin or the mucous membranes.

## Patentansprüche

1. Kosmetische oder dermatologische Zusammensetzung, umfassend eine Assoziation zwischen einer Inhibitorverbindung der Elastase der Familie der N-Acylaminoamide und wenigstens einem antifungalen Mittel oder wenigstens einem antibakteriellen Mittel, **dadurch gekennzeichnet, dass** die Inhibitorverbindung der Familie der N-Acylaminoamide eine Verbindung der Formel (I): 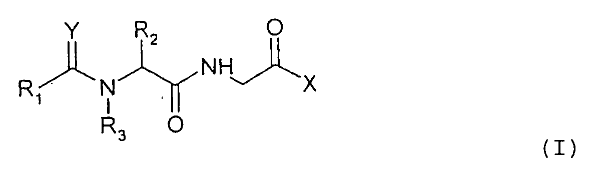 in der
- Y für Sauerstoff steht,
- der Rest R1 einen Methyl-, Ethyl-, Propyl- oder Isopropylrest darstellt, der gegebenenfalls mit einer Gruppierung
- OH oder -P(O)-(OR)₂, wobei R Methyl, Ethyl, Propyl oder Isopropyl darstellt, substituiert ist; und/oder
- der Rest R2 einen Methyl-, Ethyl-, Propyl-, Isopropyl-, n-Butyl-, tert.-Butyl- oder Isobutylrest darstellt; und/oder
- der Rest R3 eine Gruppierung darstellt, die unter denen der folgenden Formeln ausgewählt ist: 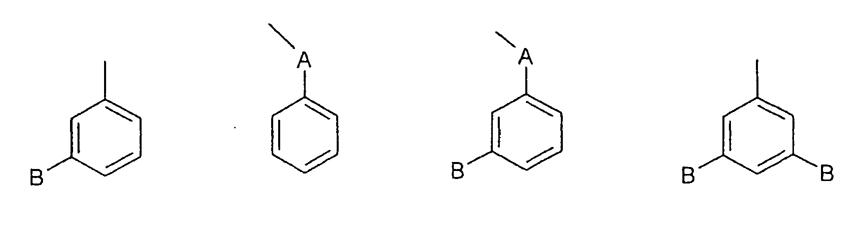 in denen der zweiwertige Rest A ein Methylen, ein Ethylen, ein Propylen ist und/oder der Rest B ein Methyl-, Ethyl-, Propyl- oder Isopropylrest ist, der mit einem oder mehreren Halogen(en) substituiert ist, insbesondere mit Chlor, Brom, Iod oder Fluor und vorzugsweise vollständig halogeniert ist (perhalogeniert ist), wie perfluoriert ist, insbesondere der Perfluormethylrest (-CF₃) ist;
- der Rest X einen Rest darstellt, ausgewählt aus -OH, -OCH₃, -OC₂H₅, -O-C₃H₇ oder -OC₄H₉,
ihre Salze mit Mineralsäuren oder organischen Säuren, ihre optischen Isomere in isolierter Form oder als racemisches Gemisch ist.

2. Zusammensetzung nach Anspruch 1, wobei die Verbindung der Formel (I) aus den folgenden Verbindungen ausgewählt ist:
- {2-[Acetyl-(3-trifluormethyl-phenyl)-amino]-3-methyl-butyrylamino}-essigsäure,
- {2-[Acetyl-(3-trifluormethyl-phenyl)-amino]-3-methyl-butyrylamino} essigsäureethylester,
- [2-(Acetyl-benzyl-amino)-3-methyl-butyrylamino]-essigsäure,
- [2-(Acetyl-benzyl-amino)-3-methyl-butyrylamino]-essigsäureethylester,
- (2-{Benzyl-[(diethoxy-phosphoryl)-acetyl]-amino}-3-methyl-butyrylamino)-essigsäureethylester.

3. Zusammensetzung nach einem der vorangehenden Ansprüche, wobei die Inhibitorverbindung der Elastase der Familie der N-Acylaminoamide in einer Menge zwischen 0,00001 und 20 Gew.-%, bezogen auf das Gesamtgewicht der Zusammensetzung und vorzugsweise zwischen 0,001 und 10 Gew.-%, vorliegt.

4. Zusammensetzung nach einem der vorangehenden Ansprüche, **dadurch gekennzeichnet, dass** das antifungale Mittel aus den folgenden Verbindungen ausgewählt ist: Imidazol-Verbindungen und ihren Derivaten; Terbinafin, Zinkpirythion, Selensulfid, Teer und seinen Verbindungen, Undecylensäure und ihren Salzen, Hydroxypyridon-Derivaten wie CICLOPIROX: 6-Cyclohexyl-1-hydroxy-4-methyl-2-(1H)-pyridon oder OCTOPIROX: 1-Hydroxy-4-methyl-6-(2,4,4,-trimethylpentyl)-2-(1H)-pyridon.

5. Zusammensetzung nach einem der vorangehenden Ansprüche, **dadurch gekennzeichnet, dass** die antifungalen Mittel in einer Konzentration zwischen 0,0001 Gew.-% und 10 Gew.-%, bezogen auf das Gesamtgewicht der Zusammensetzung, vorzugsweise zwischen 0,01 und 2 Gew.-%, bezogen auf das Gesamtgewicht der Zusammensetzung, vorliegen.

6. Zusammensetzung nach einem der vorangehenden Ansprüche, **dadurch gekennzeichnet, dass** die antibakteriellen Mittel ausgewählt sind unter der folgenden Verbindungen: Honig, einer Verbindung der Familie der Hydroxystilbene und einem halogenierten antibakteriellen Mittel und/oder einem komplexen Zucker wie Sialyllactose oder klassischen Antibiotika.

7. Zusammensetzung nach einem der vorangehenden Ansprüche, **dadurch gekennzeichnet, dass** die antibakteriellen Mittel in einer Konzentration zwischen 0,001 Gew.-% und 10 Gew.-%% des Gesamtgewichts der Zusammensetzung und vorzugsweise zwischen 0,01% und 2% vorliegen.

8. Zusammensetzung nach einem der vorangehenden Ansprüche, **dadurch gekennzeichnet, dass** sie wenigstens ein antifungales Mittel und wenigstens ein antibakterielles Mittel umfasst.

9. Zusammensetzung nach Anspruch 7, **dadurch gekennzeichnet, dass** das Gewichtsverhältnis des antifungalen Mittels zu dem antibakteriellen Mittel von 0,2 bis 10 variiert.

10. Zusammensetzung nach einem der vorangehenden Ansprüche, die sich in Form einer kosmetischen oder dermatologischen Zusammensetzung präsentiert, die zur Pflege und/oder zur Behandlung von Bereichen bestimmt ist, die ulzeriert sind oder einem kutanen Stress oder kutanen Mikrostress unterworfen waren, der insbesondere durch eine Exposition gegenüber UV und/oder Inkontaktbringen mit einem reizenden Produkt erzeugt wurde.

11. Zusammensetzung nach einem der Ansprüche 1 bis 9, die sich in der Form:
- eines Produkts zur Pflege, Behandlung, Reinigung oder zum Schutz der Haut des Gesichts oder des Körpers, hier eingeschlossen die behaarte Kopfhaut und die Schleimhäute, wie zum Beispiel eine Zusammensetzung zur Pflege (für Tag, Nacht, hydratisierend) des Gesichts oder des Körpers; einer Anti-Falten-Zusammensetzung oder einer Anti-Age-Zusammensetzung für das Gesicht; einer mattierenden Zusammensetzung für das Gesicht; einer Zusammensetzung für die irritierte Haut;
- einer Sonnenschutzzusammensetzung, einer Zusammensetzung zur künstlichen Bräunung (Selbstbräunungsmittel) oder einer Zusammensetzung zur Pflege nach dem Sonnen;
- einer Haar-Zusammensetzung und insbesondere einer Sonnenschutzcreme eines Sonnenschutzgels; einer Zusammensetzung zur Pflege der behaarten Kopfhaut, insbesondere gegen Schuppen oder für das Nachwachsen der Haare;
- eines Make-Up-Produktes für die Haut des Gesichts, des Körpers oder der Lippen, zum Beispiel eine Grundierung, eine getönte Creme, Schminke für Wangen oder Augenlider, ein lockeres oder kompaktes Puder, ein Stift gegen Augenringe, ein Deckstift, ein Lippenstift, eine Lippenpflege;
- eines buccalen Hygieneproduktes, wie zum Beispiel Zahnpasta oder Mundspülung, präsentiert.

12. Zusammensetzung nach einem der Ansprüche 1 bis 9, die sich in Form einer Zusammensetzung zur Pflege der Haut des Gesichts, des Anti-Falten-Typs oder Anti-Age-Typs oder in Form einer Sonnenschutzzusammensetzung oder einer Zusammensetzung zur Anwendung nach dem Sonnen präsentiert.

13. Verfahren zur kosmetischen Behandlung der Haut des Körpers oder des Gesichts, hier eingeschlossen die behaarte Kopfhaut, bei dem man eine kosmetische Zusammensetzung, wie sie in einem der Ansprüche 1 bis 12 definiert ist, auf die Haut oder die Schleimhäute anwendet.
